# EUROPEAN PATENT APPLICATION

(11) **EP 0 945 508 A1**
(43) Date of publication of application: **29.09.1999**
(21) Application number: 98301988.6
(22) Date of filing: 17.03.1998
(51) Int. Cl.: C12N 15/29, C12N 15/82, A01N 65/00, C07K 14/415, C07K 14/81, C12N 1/20, C12N 5/10, C12P 21/02

(54) **The insect-resistant use of sweet potato sporamin gene and method for controlling pests using the gene**

(71) Applicant: NATIONAL SCIENCE COUNCIL, Taipei (TW)
(72) Inventor: Yeh, Kai-Wun, Taipei (TW); Lin, Mei-In, Taipei (TW); Tuan, Shu-Jen, Taipei (TW); Chen, Yih-Ming, Taipei (TW); Lin,Chu-Yung, Taipei (TW); Kao, Suey-Sheng, Taipei (TW)
(74) Representative: Woods, Geoffrey Corlett

(57) **Abstract**

The use of sweet potato sporamin gene in controlling pests is provided. The gene with insect-resistance is inserted into an appropriate vector, and transformed into plants to enhance the insect-resistance so as to attain the purpose of controlling pests.

SEQUENCE LISTING

(1) GENERAL INFORMATION:

(i) APPLICANT:
(A) NAME: NATIONAL SCIENCE COUNCIL
(B) STREET: 106 Sec. 2, Ho-Ping E. Road
(C) CITY: Taipei
(E) COUNTRY: Taiwan, Republic of China

(ii) TITLE OF INVENTION: THE INSECT-RESISTANT USE OF SWEET POTATO
SPORAMIN GENE AND METHOD FOR CONTROLLING PESTS USING THE
GENE

(iii) NUMBER OF SEQUENCES: 1

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 98301988.6

(2) INFORMATION FOR SEQ ID NO: 1:

(i) SEQUENCE CHARACTERISTICS:
(A) LENGTH: 899 base pairs
(B) TYPE: nucleic acid
(C) STRANDEDNESS: double
(D) TOPOLOGY: linear

(ii) MOLECULE TYPE: cDNA

(xi) SEQUENCE DESCRIPTION: SEQ ID NO: 1:

AATTAAACAT CATTACCTCT TCGCTTTCTC CCAATTAAGG TTGTCATCTG CCACCATGAA 60

AGCCCTCACA CTGGCACTCT TCTTAGCCCT TTCCCTCTAT CTCCTCCCCA ATCCCGCCCA 120

TTCCAGGTTC AATCCCATCC GCCTCCCCAC CACACACGAA CCCGCCTCCT CTGAAACTCC 180

AGTACTGGAC ATCAACGGCG ACGAGGTCCG CGCCGGCGGG AACTACTACA TGGTCTCCGC 240

CATATGGGGA GCCGGCGGGG GAGGGCTAAG ACTCGCCCAC TTGGACATGA TGTCCAAATG 300

CGCCAGCGAC GTCATCGTAT CCCCCAACGA CTTAGACAAC GGCGACCCCA TCACCATCAC 360

GCCGGCGACG GCCGACCCGG AATCCACCGT GGTCATGGCG TCGACGTACC AGACTTTCCG 420

GTTCAACATC GCCACCAACA AGCTCTGCGT GAACAACGTG AACTGGGGAA TCCAGCACGA 480

CAGCGCGTCC GGGCAGTATT TCCTGAAAGC CGGCGAGTTT GTGTCCGACA ATAGCAACCA 540

GTTCAAGATT GAGCTGGTGG ATGCCAACCT TAACTCCTAC AAACTCACTT ACTGTCAGTT 600

CGGCTCCGAT AAATGCTACA ACGTCGGCAG ATTCCACGAC CACATGTTGA GGACCACGCG 660

TTTGGCTCTC TCCAATTCTC CCTTCGTTTT TGTCATCAAA CCTACCGATG TGTAATGTAA 720

CACTGAAAAG CGCCGGTTAT GAGGTTGCAT GGTAGCTATG CAACGTTGCC ACTTTGACAA 780

CGTTGTACGT GTAAGAATAA ACATGCAACA AATCCGAGCT GGTATGGTTG TGTAAATCCT 840

AAATAAATCC GAAGAAATAA TAAGGATAAA ATATTATCCT GTGTTTGTTT TAATTCTCC 899

## Description

### Field of Invention

The present invention provides the insect resistant use of sweet potato sporamin gene, and a method for controlling pests using the gene.

### Background of Invention

In the past few years, according to the development of genetic engineering techniques, the introduction of genes encoding insect-resistant substances into plants by transgenic techniques can enhance the insect-resistance of the transgenic plants. The insect-resistant substances include, such as toxic crystalline protein, protease inhibitor and the similar produced by *Bacillus thuringiensis*.

Sporamin is a storage protein enriched in the tuberous roots of sweet potato, which was first purified by Maeshima et al. (Maeshima *et al*., "Characterization of major proteins in sweet potato tuberous roots." *Phytochemistry*, Vol. 124. pp. 1899-1902, 1985). The amount of Sporamin is about 60% to 80% of the total soluble proteins of sweet potato. Most sporamin is present in tuberous roots, little in other tissues and organs (Hattori *et al*., "High-level expression of tuberous root storage protein genes from sweet potato in stems of plantlets grown in vitro on sucrose medium." *Plant Mol. Biol*. Vol. 14, pp. 595-604, 1990). In 1989, the sporamin gene was isolated from the cDNA library of tuberous roots of sweet potato (Hattori *et al*.. "Structural relationship among the members of multigene family coding for the sweet potato tuberous roots storage proteins." *Plant Mol. Biol.* Vol. 13, pp. 563-572, 1989).

In addition to the function of storing nitrogen source, it is found from the analysis of the amino acid sequence of sporamin predicted from the cDNA thereof that there is certain homology to Kunitz type trypsin inhibitors of Leguminosae plants (Hattori *et al*., "Sucrose-induced express of genes coding for the tuberous root storage protein sweet potato in leaves and petioles." *Plant Cell Physiol*. Vol. 32, pp. 79-86, 1991). However, Hattori *et al*. suggested that sporamin is not active as a trypsin inhibitor (Hattori *et al*., 1989).

Moreover, the relationship between sweet potato sporamin gene and trypsin inhibitor activities was discussed by Chen, Jen-chih and Yeh, Kai-Wun in "The research in the genome of sweet potato," Dept. of Botany, National Taiwan University, Master thesis. pp. 1-122 (1994). The article that sporamin might possess trypsin inhibitor activity.

The purpose of the present invention is to locate appropriate genes which can be transfected into plants so as to provide a method of controlling pests. According to the present invention, it is found that sporamin gene can be used in controlling pests, so it is possible to develop a method for controlling pests.

### Summary of Invention

The object of the present invention is to provide the use of sweet potato sporamin gene in controlling pests.

The further object of the present invention is to provide a method for controlling pests, in which the sweet potato sporamin gene has been transfected into a plant of tobacco model plants to enhance the insect- resistance of said plant.

Still the further object of the present invention is to provide a transformed vector, in which the sweet potato sporamin gene is inserted into an appropriate vector, thereby the sweet potato sporamin gene will be transformed into a plant to enhance the insect resistance of the plant.

The present invention also provides a transformed bacterium, which is used as a vehicle to transfer the sweet potato sporamin gene into a plant to enhance the insect resistance of the plant.

### Description of Figures

Fig. 1. Full length cDNA sequence of sporamin, in which the initiation sequence for translation [ATG] and the polyadenylation signal sequence [AATAAA] are underlined.
Fig. 2. Construction of Plasmid pBI 121, in which the full length cDNA sequence of sporamin was inserted at the *XbaI/BamHI* site in pBI 121.
Fig. 3. Northern blotting of the transformed *Agrobacterium tumefaciens* wherein TIA cDNA was used as gene probe. In the figure, C represents untransformed Agrobacterium LBA 4404 plasmid DNA, P represents a vector pBI 121 with sporamin gene insert, and 1- 10 represent the plasmid DNAs from transformed Agrobacterium.
Fig. 4. Analysis of trypsin inhibitor activity of the transgenic plant's protein. In the figure,. W represents the leaf protein extracted from a wild-type plant which is a nontransgenic plant, 1-3 and 6-8 represent the leaf protein extracted from transgenic plants. The transparent region shows the place at which trypsin inhibitor activity is present. The transgenic plants 1, 2 and 8 were the most active.
Fig. 5. The damage on leaves tranformed with various strains of *Spodoptera litura* (tobacco cutworm) in the insect-resistance test.

### The detailed description of the invention

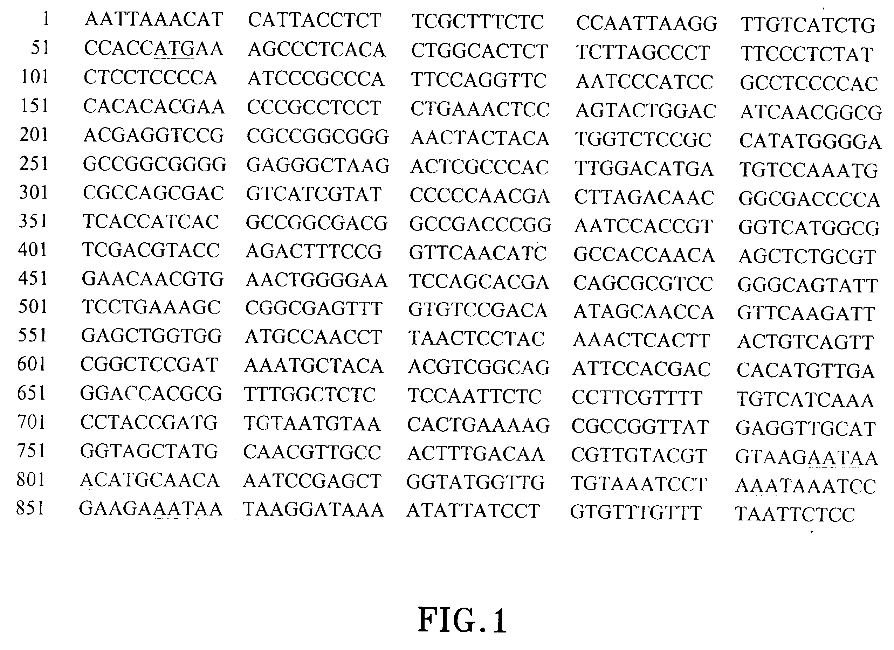

The present invention provides a novel use of sweet potato sporamin gene. As mentioned herein, the sweet potato sporamin gene refers to the full length cDNA sequence of said gene, as shown in Fig. 1. The cDNA of sporamin gene is 0.93 kb in full length. A preferred embodiment of the present invention is a full length cDNA sequence isolated from sweet potato (*Ipomoea batatas* (L.) Lam Tainong 57) tuberous root, designated spTi- 1, under accession number U17333 with GenBank (Yeh *et al*., 1994).

The sweet potato sporamin gene has been proven by the inventor to have trypsin inhibitor activity. Therefore, a study on the expression of tobacco model plants transformed with sporamine gene was conducted to evaluate the insect resistance of the transformed plants so as to attain the purpose of controlling pests.

As a vector for transfecting the sweet potato sporamin gene into a bacterium, the binary vectors suitable for plant transformation, such as plasmid pBI 121, pBin , pZp and the like are preferable.

The transformation vectors described above are used for transfecting sporamin gene into plants mediated through an appropriate bacterium as vehicle. A preferable embodiment of the present invention is *Agrobacterium tumefaciens* LBA 4404, which is expressed under appropriate control and only contains disarmed Ti plasmid pAL 4404.

According to the invention, the method of controlling pests comprises transforming a plant with sporamin gene thereby said plant has the properties of sporamin, i.e. exhibiting trypsin inhibitor activity, so as to attain the purpose of controlling pests. In an embodiment of the present invention, sporamin gene was inserted into an appropriate vector, an appropriate bacterium was transformed with the resulting vector, and said transformed bacterium as vehicle and a plant were co-incubated so as to introduce the sporamin gene into said plant. As the result, the insect resistance of said transformed plant was enhanced.

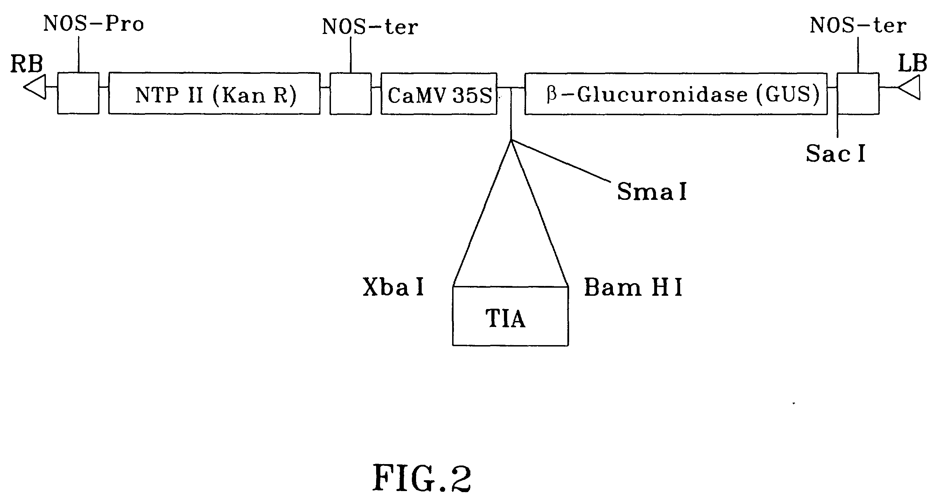

According to the present invention, a transformation vector TIA :: pBI 121 was prepared. The construction of Plasmid pBI 121 is shown in Fig. 2. It was cut at the *XbaI/BamHI* site in pBI 121 where sporamin gene was inserted into.

According to the present invention, a transformant bacterium was prepared by transforming *Agrobacterium tumefaciens* LBA 4404 directly with TIA :: pBI 121. Because the vector pBI 121 carries neomycin transferase gene, Kanamycin can be used to select the Agrobacteria or leaves which have successfully been transformed. Among the transformed *Agrobacterium tumefaciens* according to the invention, one of the representative transformants, tBA, has been deposited in the Food Industry Research and Development Institute, Hsin-Chu, Taiwan, ROC, on 27 January 1997 under accession number of CCRC 910072.

The following examples are provided by way of illustration and not by way of limitation.

### Example 1

### The construction of a vector inserted with sweet potato sporamin gene

A full-length cDNA sequence of sweet potato sporamin gene was isolated from a sweet potato tuberous cDNA library, which was designated spTi-1, under accession number U17333 with GenBank (Chen, 1994). This cDNA fragment was inserted at a *XbaI/BamHI* site of the binary vector pBI 121 (ClonTech Co.). The plasmid was expressed under the control of 35S CaMV promoter, and the 3'-Nos terminator. According to standard molecular procedure, the constructed sequence was determined to identify the precise orientation (Sambrook et al., Molecular Cloning : A Laboratory Manual, Cold Spring Harbor Laboratory Press., 1989). The construction of that vector is shown in Fig. 2, and designated TIA :: pBI 121.

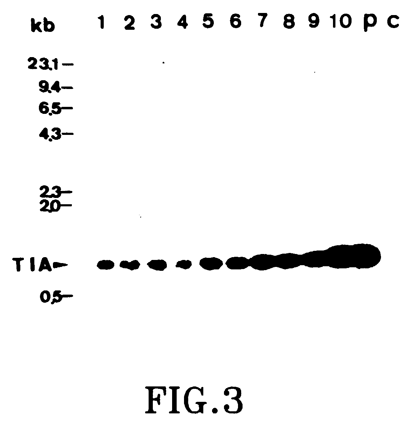

The *Agrobacterium tumefaciens* LBA 4404 was directly transformed with the resulting vector (Höfgen & Willmitzer, Nucleic Acids Research Vol. 16, pp. 9877-9878, 1988). Ten transformants, L1 to L 10 were obtained. One of the representative transformants is tBA, which has been deposited in the Food Industry Research and Development Institute. The integrity of the plasmid was determined by Southern blot hybridization. The result is shown in Fig. 3, which demonstrates that said *Agrobacterium tumefaciens* LBA 4404 has been transformed with sporamin gene.

### Example 2

### Tranformation of Tobacco leaf-discs

Tobacco (*Nicotiana tabaccum* cv. W38) model plants were transfected by sporamin gene mediated through a strain of *A. tumefaciens Agrobacterium*, according to the protocol disclosed by Horsch et al. (Horsch et al., Science Vol. 227, pp. 1229-1231, 1985). The culture of *A. tumefaciens* with sporamin gene insert obtained from Example 1 was co-incubated with tobacco leaf discs for 3 days in the dark at 28 °C. Then, after rinsed with sterile water several times, the leaf discs were subsequently cultured in petri dishes containing Kanamycin at 28 °C, 16 hours in the light for selecting the regenerating seedlings which have successfully been transformed. After 2 to 3 weeks, shoots of said seedings were excised and transferred to another medium for root-induction. Three to four week-old plantlets were transferred to pots, and grown under a conditioned growth chamber, at 28 ± 2 °C , 16 h light/day, 75% relative humidity. Finally, the expression of TIA gene and the production of protein with trypsin inhibitor activity in the transformants were determined by Western blot hybridization.

### Example 3

### Assay for inhibitor activity

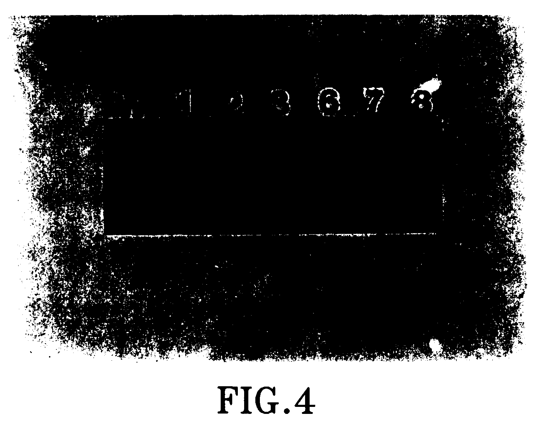

The trypsin inhibitor activity of transgenic tobacco protein was determined. The electrophoresis of the proteins extracted from transgenic tobacco leaves was carried out on SDS-PAGE. Then, the electrophoresis gel was added with an appropriate trypsin solution, and incubated at room temperature for 30-40 minutes. The gel was rinsed to remove excess trypsin, and incubated at room temperature for 2-12 hours after adding trypsin substrate (N-acetyl-DL-phenylalanine β-naphthyl ester (APNE)) solution and dye solution (O-dianisidine, tetrazoitize solution). The reaction was stopped by adding acetic acid. The electrophoresis gel with any transparent region was examined. Said transparent region results from the existence of trypsin inhibitor inactivating the reaction of trypsin and substrate. Therefore, the gel carrying trypsin inhibitor cannot be stained. In other words, the presence of the transparent region means that said protein has trypsin inhibitor activity. The results were shown in Fig. 4.

### Example 4

### Bioassay for insect-resistance of transgenic tobacco plants

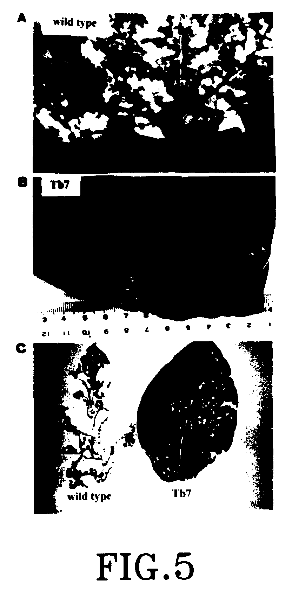

Late first or Early second instar larvae of *Spodoptera litura* (tobacco cutworm) were fed with sporamin gene-transformed tobacco leaves individually, and incubated in a growth chamber (at temperature of 25 ± 1 °C, 70 ± 5% relative humidity). The mortality of larvae, the average body weight of survival larvae, and the damage ratio of leaf area by larvae were observed and recorded. Each treatment was conducted for 30 larvae, and untransformed tobacco leaves was used as Control. The results were listed in Table 1. As compared with Control, the death of larvae resulted from the transgenic plants. In particular for Tb 7 plant, the survival rate of larvae was only 6.7%, and the average weight of survival larvae is 1/5 of that of Control. Further, the damage leaf area of wild tobacco (Control) and sporamin gene-transformed tobacco (Tb 7) were shown in Fig. 5. In view of the leaf entirety of Tb 7 plant, it was suggested that sporamin gene-transformed plants exhibit a better resistance against *Spodoptera litura* (tobacco cutworm).

**Table 1**

| Results of insect-resistance test in transgenic tobacco | | | |
|---|---|---|---|
| | % Insect Survival | Average Body Weight (mg) | Damage Area /lar. (cm²) |
| Wild type | 100.0 | 33.4 ± 4.4 | 5.5 |
| Tb-1 | 43.5 | 15.7 ± 4.2 | 4.3 |
| Tb-2 | 77.8 | 17.8 ± 5.9 | 2.6 |
| Tb-3 | 96.4 | 12.0 ± 3.1 | 1.6 |
| Tb-6 | 96.8 | 24.6 ± 1.8 | 6.4 |
| Tb-7 | 6.7 | 6.1 ± 0.8 | 0.8 |
| Tb-8 | 91.3 | 16.3 ± 3.3 | 2.2 |

### Figure Legends

- Fig. 1: Alignment of the full length sporamin DNA
- Fig. 2: Construction of Plasmid TIA:pBI 121
- Fig. 3: Southern blot hybridization of transformed Agrobacterium, wherein TIA cDNA was used as probe.
- Fig. 4: An alysis of trypsin inhibitor activity of proteins expressed in the transgenic plants.
- Fig. 5: Insect-resistance bioassay: the damage of tobacco leaves by *Spodoptera litura* (tobacco cutworm).

### Further Description of the Invention

### Polynucleotides

A polynucleotide of the invention may consist essentially of the nucleotide sequence of Fig 1 (SEQ ID No. 1) or of a coding sequence within the sequence of SEQ ID No. 1

Preferably, a polynucleotide of the invention is capable of hybridising selectively with the coding sequence of SEQ ID No. 1, to a coding sequence within it or to the sequence complementary to one of those sequences. Polynucleotides of the invention include variants of the coding sequence of SEQ ID No. 1 that encode sporamin polypeptides of the invention. Typically, a polynucleotide of the invention is a contiguous sequence of nucleotides which is capable of selectively hybridising to the sequence of SEQ ID. No. 1, to a coding sequence within SEQ ID No. 1 or to the complement of one of those sequences.

A polynucleotide of the invention can hybridise to coding sequence of SEQ ID No. 1 at a level significantly above background. Background hybridisation may occur, for example, because of other cDNAs present in a cDNA library. The signal level generated by the interaction between a polynucleotide of the invention and the coding sequence of SEQ ID No. 1 is typically at least 10 fold, preferably at least 100 fold, as intense as interactions between other polynucleotides and the coding sequence of SEQ ID No. 1. The intensity of interaction may be measured, for example, by radiolabelling the probe, e.g. with ³²P. Selective hybridisation is typically achieved using conditions of medium to high stringency (for example 0.03M sodium chloride and 0.03M sodium citrate at from about 50°C to about 60°C). If high stringency is required, condition such as 0.2 x SSC at 60°C can be used; for lower stringency, conditions such as 2 x SSC at 60°C can be used.

A nucleotide sequence capable of selectively hybridising to the DNA coding sequence of SEQ ID No. 1 or a coding sequence within SEQ ID No. 1 or to the sequence complementary to one of those coding sequences will be generally at least 70%, preferably at least 80 or 90% and more preferably at least 95%, 98% or 99%, homologous to the sequence of SEQ ID No. 1, or a coding sequence within SEQ ID No. 1, or the complement of one of those sequences over a region of at least 20, preferably at least 30, for instance at least 40, 60 or 100 or more contiguous nucleotides.

Any combination of the above mentioned degrees of homology and minimum sizes may be used to define polynucleotides of the invention, with the more stringent combinations (i.e. higher homology over longer lengths) being preferred. Thus for example a polynucleotide which is at least 90% homologous over 25, preferably over 30 nucleotides forms one aspect of the invention, as does a polynucleotide which is at least 95% homologous over 40 nucleotides.

Polynucleotides of the invention may comprise DNA or RNA. They may also be polynucleotides which include within them synthetic or modified nucleotides. A number of different types of modification to polynucleotides are known in the art. These include methylphosphonate and phosphorothioate backbones, addition of acridine or polylysine chains at the 3' and/or 5' ends of the molecule. For the purposes of the present invention, it is to be understood that the polynucleotides described herein may be modified by any method available in the art. Such modifications may be carried out in order to enhance the *in vivo* activity or lifespan of polynucleotides of the invention.

Polynucleotides of the invention may be used to produce a primer, e.g. a PCR primer, a primer for an alternative amplification reaction, a probe e.g. labelled with a revealing label by conventional means using radioactive or non-radioactive labels, or the polynucleotides may be cloned into vectors. Such primers, probes and other fragments will preferably be at least 10, preferably at least 15 or 20, for example at least 25, 30 or 40 nucleotides in length.

Polynucleotides such as a DNA polynucleotide and primers according to the invention may be produced recombinantly, synthetically, or by any means available to those of skill in the art. They may also be cloned by standard techniques. The polynucleotides are typically provided in isolated and/or purified form.

In general, primers will be produced by synthetic means, involving a stepwise manufacture of the desired nucleic acid sequence one nucleotide at a time. Techniques for accomplishing this using automated techniques are readily available in the art.

Genomic clones corresponding to the cDNA of SEQ ID No. 1 containing, for example, introns and promoter regions are also aspects of the invention and may also be produced using recombinant means, for example using PCR (polymerase chain reaction) cloning techniques, starting with genomic DNA from a plant cell.

Although in general the techniques mentioned herein are well known in the art, reference may be made in particular to Sambrook *et al*, 1989, Molecular Cloning: a laboratory manual.

Polynucleotides which are not 100% homologous to the sequences of the present invention but fall within the scope of the invention can be obtained in a number of ways.

Other allelic variants of the sweet potato sequence of SEQ ID No. 1 may be obtained for example by probing genomic DNA libraries made from a range of wheat cells, using probes as described above.

In addition, other plant homologues of SEQ ID No. 1 may be obtained and such homologues and fragments thereof in general will be capable of selectively hybridising to the coding sequence of SEQ ID No. 1, a coding sequence within SEQ ID No.1 or the complement of one of those sequences. Such sequences may be obtained by probing cDNA or genomic libraries from other plant species with probes as described above. Degenerate probes can be prepared by means known in the art to take into account the possibility of degenerate variation between the DNA sequences of SEQ ID No. 1 and the sequences being probed for under conditions of medium to high stringency (for example 0.03M sodium chloride and 0.03M sodium citrate at from about 50°C to about 60°C).

Allelic variants and species homologues may also be obtained using degenerate PCR which will use primers designed to target sequences within the variants and homologues encoding likely conserved amino acid sequences. Likely conserved sequences can be predicted from aligning the amino acid encoded by SEQ ID No. 1 with that of other similar sporamin sequences. The primers will contain one or more degenerate positions and will be used at stringency conditions lower than those used for cloning sequences with single sequence primers against known sequences.

Alternatively, such polynucleotides may be obtained by site directed mutagenesis of SEQ ID No. 1 or allelic variants thereof. This may be useful where, for example, silent codon changes are required to sequences to optimise codon preferences for a particular host cell in which the polynucleotide sequences are being expressed. Other sequence changes may be desired in order to introduce restriction enzyme recognition sites, or to alter the property or function of the polypeptides encoded by the polynucleotides.

The invention further provides double stranded polynucleotides comprising a polynucleotide of the invention and its complement.

Polynucleotides, probes or primers of the invention may carry a revealing label. Suitable labels include radioisotopes such as ³²P or ³⁵S, enzyme labels, or other protein labels such as biotin. Such labels may be added to polynucleotides, probes or primers of the invention and may be detected using techniques known *per se*.

The present invention also provides polynucleotides encoding the polypeptides of the invention described below. Because such polynucleotides will be useful as sequences for recombinant production of polypeptides of the invention, it is not necessary for them to be selectively hybridisable to the coding sequence of sequence SEQ ID No. 1, although this will generally be desirable. Otherwise, such polynucleotides may be labelled, used, and made as described above if desired. Polypeptides of the invention are described below.

Particularly preferred polynucleotides of the invention are those of SEQ ID No. 1 and coding sequences within SEQ ID No. 1. In particular, there is an initiator codon, ATG at positions 56 to 58 of SEQ ID No.1 and an in-frame stop codon, TAA, at positions 713 to 715. It is preferred that polynucleotides of the invention comprise the coding sequence defined by those two codons, i.e. the sequence beginning with nucleotide 56 and ending with nucleotide 712 (the final coding nucleotide of this sequence) or 715 (including the stop codon). Also preferred are polynucleotides encoding fragments of this sequence that encode polypeptides of the invention as defined below.

The invention also provides sequences which are degenerate to the coding sequences described above, as a result of the degeneracy of the genetic code. As the skilled person will appreciate, the genetic code is "degenerate" in the sense that certain amino acids can be encoded by more than one triplet codon of nucleotides. Thus, different nucleotide sequences can give encode the same amino acid sequence. Such degenerate nucleotide sequences are embodiments of the invention.

### Polypeptides

A polypeptide of the invention consists essentially of an amino acid sequence encoded by a polynucleotide of the invention.

The polypeptides of the invention are sporamin polypeptides. The Inventor has found that the sweet potato sporamin polypeptide encoded by the sequence of SEQ ID No. 1 has trypsin inhibitor activity. The Inventor has also found that this polypeptide confers insect resistance on plants; in particular it confers resistance to tobacco cutworm on tobacco plants.

Preferably, the polypeptides of the invention have trypsin inhibitor activity. This can be assayed by techniques known in the art, for example as described in Example 3. More preferably, the polypeptide has the ability to confer insect resistance on plants, when applied to the plants or when produced *in situ* by the plants' expression of a polynucleotide of the invention.

This can be assayed by any means known it the art. One means of assaying a polypeptide's ability to confer insect resistance on plants is to transform a plant tissue (e.g a tobacco leaf disc as in Example 2) with a polynucleotide, vector or chimeric gene of the invention, regenerate a transgenic plant from the tissue and determine the degree to which the transgenic plant is insect-resistant by comparing the mortality of insects (e.g. tobacco cutworm for tobacco) on transgenic plants and on no-transgenic control plants

In particular, a polypeptide of the invention may comprise:
(a) the polypeptide sequence encode by nucleotides 56 to 712 of SEQ ID No. 1;
(b) an allelic variant or species homologue thereof; or
(c) a polypeptide at least 70, 80, 90, 95, 98 or 99% homologous to (a) or (b).

An allelic variant (b) will be a variant which will occur naturally in a plant and which will function in a substantially similar manner to the polypeptide of (a). Similarly, a species homologue (c) of polypeptide (a) will be the equivalent polypeptide which occurs naturally in another plant species. Such a homologue may occur in plants other than sweet potato. Within any one species, a homologue may exist as several allelic variants, and these will all be considered homologues of the polypeptide of (a)

Allelic variants (b) and species homologues (c) can be obtained by following the procedures described herein for the production of the polypeptides (a) and performing such procedures on a suitable cell source. It will also be possible to use a probe as defined above to probe libraries made from plant cells in order to obtain clones encoding the allelic or species variants. The clones can be manipulated by conventional techniques to generate a polypeptide of the invention which can then be produced by recombinant or synthetic techniques known *per se*.

A polypeptide of the invention is preferably at least 70% homologous to the polypeptide of (a), more preferably at least 80 or 90% and more preferably still at least 95%, 97% or 99% homologous thereto over a region of at least 20, preferably at least 30, for instance at least 40, 60 or 100 or more contiguous amino acids. Methods of measuring protein homology are well known in the art and it will be understood by those of skill in the art that in the present context, homology is calculated on the basis of amino acid identity (sometimes referred to as "hard homology").

The sequence of the polypeptide of (a) and of allelic variants and species homologues can thus be modified to provide polypeptides of the invention.

Amino acid substitutions may be made, for example from 1, 2 or 3 to 10, 20 or 30 substitutions. The modified polypeptide remains a sporamin polypeptide, as defined herein. Conservative substitutions may be made, for example according to the following Table. Amino acids in the same block in the second column and preferably in the same line in the third column may be substituted for each other.

| | | |
|---|---|---|
| ALIPHATIC | Non-polar | G A P |
| | | I L V |
| | Polar-uncharged | C S T M |
| | | N Q |
| | Polar-charged | D E |
| | | K R |
| AROMATIC | | H F W Y |

Polypeptides of the invention also include fragments of the above-mentioned full length polypeptides and variants thereof, which fragments typically remain sporamin polypeptides as defined herein.

Polypeptides of the invention may be in a substantially isolated form. It will be understood that the polypeptide may be mixed with carriers or diluents which will not interfere with the intended purpose of the polypeptide and still be regarded as substantially isolated. A polypeptide of the invention may also be in a substantially purified form, in which case it will generally comprise the polypeptide in a preparation in which more than 90%, e.g. 95%, 98% or 99% of the polypeptide in the preparation is a polypeptide of the invention.

Polypeptides of the invention may be chemically modified, e.g. post-translationally modified. For example, they may be glycosylated or comprise modified amino acid residues. Such modified polypeptides and proteins fall within the scope of the terms "polypeptide" of the invention.

### Vectors and chimeric genes

Polynucleotides of the invention can be incorporated into a recombinant replicable vector. The vector may be used to replicate the nucleic acid in a compatible host cell. Thus in a further embodiment, the invention provides a method of making polynucleotides of the invention by introducing a polynucleotide of the invention into a replicable vector, introducing the vector into a compatible host cell, and cultivating the host cell under conditions which bring about replication of the vector. The vector may be recovered from the host cell. Suitable host cells are described below in connection with expression vectors. Bacterial cells, especially *Escherichia coli*, and *Agrobacterium tumefaciens* are preferred.

### Expression vectors

Preferably, a polynucleotide of the invention in a vector is operably linked to regulatory sequences capable of effecting the expression of the coding sequence by the host cell, i.e. the vector is an expression vector. Such expression vectors can be used to express the polypeptides of the invention.

The term "operably linked" refers to a juxtaposition wherein the components described are in a relationship permitting them to function in their intended manner. A regulatory sequence "operably linked" to a coding sequence is positioned in such a way that expression of the coding sequence is achieved under conditions compatible with the regulatory sequences.

Such vectors may be introduced into a suitable host cell to provide for expression of a polypeptide or polypeptide fragment of the invention, as described below.

The vectors may be for example, plasmid, virus or phage vectors provided with an origin of replication, preferably a promoter for the expression of the said polynucleotide and optionally an enhancer and/or a regulator of the promoter. For expression in plant cells, one preferred enhancer is the Tobacco etch virus (TEV) enhancer. A terminator sequence may also be present, as may a polyadenylation sequence. The vectors may contain one or more selectable marker genes, for example an ampicillin resistance gene in the case of a bacterial plasmid or a neomycin resistance gene (e.g. nptI or nptII) or methotrexate resistance gene for a plant vector. Vectors may be used *in vitro*, for example for the production of RNA or used to transfect or transform a host cell. The vector may also be adapted to be used *in vivo*, for example for generation of transgenic plants of the invention. Preferred vectors include binary vectors, especially pBI 122.

So far as plasmid vectors are concerned, plasmids derived from the Ti plasmid of *Agrobacterium tumefaciens* are especially preferred, as are plasmids derived from the Ri plasmid of *Agrobacterium rhizogene*s.

A further embodiment of the invention provides host cells transformed or transfected with the vectors for the replication and expression of polynucleotides of the invention. The cells will be chosen to be compatible with the said vector and may for example be prokaryotic (bacterial), plant, yeast, insect or mammalian cells, bacterial and plant cells being preferred. Cells of *Agrobacterium tumefacien*s are especially preferred

Promoters and other regulatory elements may be selected to be compatible with the host cell for which the expression vector is designed.

Promoters suitable for use in plant cells may be derived, for example, from plants or from bacteria that associate with plants or from plant viruses. thus, promoters from *Agrobacterium spp*. including the nopaline synthase (nos), octopine synthase (ocs) and mannopine synthase (mas) promoters are preferred. Also preferred are plant promoters such as the ribulose bisphosphate small subunit promoter (rubisco ssu), and the phaseolin promoter. Also preferred are plant viral promoters such as the cauliflower mosaic virus (CAMV) 35S and 19S promoters.

Depending on the pattern of expression desired, promoters may be constitutive or inducible. For example, strong constitutive expression in plants can be obtained with the CAMV 35S or rubisco (ssu) promoters. Also, tissue-specific or stage-specific promoters may be used to target expression of polypeptides of the invention to particular tissues in a transgenic plant or to particular stages in its development.

Especially where expression in plant cells is desired, other regulatory signals may also be incorporated in the vector, for example a terminator and/or polyadenylation site. One preferred terminator is the nos terminator although other terminators functional is the nos terminator in plant cells may also be used. It should be noted that the sequence of SEQ ID No. 1 contains three polyadenylation sites

Additionally, sequences encoding secretory signals or transit peptides may be included. On expression, these elements direct secretion from the cell or target the polypeptide of the invention to a particular location within the cell. For example, sequences may be added to target the expressed polypeptide to the nucleus or plastids (e.g. chloroplasts) of a plant cell.

### Chimeric Genes

The invention also provides chimeric genes suitable for securing the expression of polypeptides of the invention in a host cell, preferably a plant cell. These comprise a polynucleotide of the invention, operably linked to regulatory sequences that allow its expression in a host cell, preferably a plant cell.

Typically, therefore, a chimeric gene comprises the following elements in 5' to 3' orientation: a promoter functional in a host (preferably plant) cell, as defined above, a polynucleotide of the invention and a terminator functional in said cell, as defined above. Other elements, for example an enhancer, may also be present. These chimeric genes may be incorporated into vectors, as defined above.

### Expression in host cells

Expression vectors of the invention may be introduced into host cells using conventional techniques including calcium phosphate precipitation, DEAE-dextran transfection, or electroporation. For plant cells, preferred transformation techniques include electroporation of plant protoplasts, transformation by *Agrobacterium tumefaciens* and particle bombardment. Particle bombardment is particularly preferred for transformation of monocot cells.

Expression in the host cell may be transient although, preferably, integration of the polynucleotide or chimeric gene of the invention into the cell's genome is achieved.

Suitable cells include cells in which the above-mentioned vectors may be expressed. These include microbial cells such as bacteria such as *E. coli*, plant cells, mammalian cells such as CHO cells, COS7 cells or Hela cells, insect cells or yeast such as *Saccharomyces*. Bacterial and plant cells are preferred.

Cell culture will take place under standard conditions. Commercially available cultural media for cell culture are widely available and can be used in accordance with manufacturers' instructions.

### Processes for production of polypeptides

The invention provides processes for the production of polypeptides and dimeric proteins of the invention by recombinant means.

Processes for the production of polypeptides of the invention may comprise:
(a) cultivating a transformed cell as defined above under conditions that allow the expression of the polypeptide;
   and preferably
(b) recovering the expressed polypeptide.

### Methods of producing transgenic plant cells, plant parts and tissues, plants and seeds of the invention

Transgenic plant cells, plant parts and tissues, plants and seeds of the invention are transgenic in the sense that they have at least one polynucleotide of the invention introduced into them.

The invention provides a method of obtaining a transgenic plant cell comprising transforming a plant cell with an expression vector of the invention to give a transgenic plant cell.

Any suitable transformation method may be used, for example the transformation techniques described herein. Suitable transformation techniques include electroporation of plant protoplasts, transformation by *Agrobacterium tumefacien*s and particle bombardment. Transformation by *Agrobacterium tumefaciens* is particularly preferred.

The cell may be in any form. For example, it may be an isolated cell, e.g. a protoplast, or it may be part of a plant tissue, e.g. a callus, or a tissue excised from a plant, or it may be part of a whole plant. Transformation may thus give rise to a chimeric tissue or plant in which some cells are transgenic and some are not.

Preferably, integration of a polynucleotide or chimeric gene of the invention into the cell's genome is achieved.

The thus obtained cell may be regenerated into a transgenic plant by techniques known in the art. These may involve the use of plant growth substances such as auxins, giberellins and/or cytokinins to stimulate the growth and/or division of the transgenic cell. Similarly, techniques such as somatic embryogenesis and meristem culture may be used. Regeneration procedures will typically involve the selection of transformed cells by means of marker genes.

In many such techniques, one step is the formation of a callus, i.e. a plant tissue comprising expanding and/or dividing cells. Such calli are a further aspect of the invention as are other types of plant cell cultures and plant parts. Thus, for example, the invention provides transgenic plant tissues and parts, including embryos, meristems, seeds, shoots, roots, stems, leaves and flower parts. These may be chimeric in the sense that some of their cells are transgenic and some are not.

The regeneration step gives rise to a first generation transgenic plant. The invention also provides methods of obtaining transgenic plants of further generations this first generation plant. These are known as progeny transgenic plants. progeny plants of second, third fourth, fifth, sixth and further generations may be obtained from the first generation transgenic plant by any means known in the art.

Thus, the invention provides a method of obtaining a transgenic progeny plant comprising obtaining a second-generation transgenic progeny plant from a first-generation transgenic plant of the invention, and optionally obtaining transgenic plants of one or more further generations from the second-generation progeny plant thus obtained.

Such progeny plants are desirable because the first generation plant may not have all the characteristics required for cultivation. For example, for the production of first generation transgenic plants, a plant of a taxon that is easy to transform and regenerate may be chosen. It may therefore be necessary to introduce further characteristics in one or more subsequent generations of progeny plants before a transgenic plant more suitable for cultivation is produced.

Progeny plants may be produced from their predecessors of earlier generations by any known technique. In particular, progeny plants may be produced by:
obtaining a transgenic seed from a transgenic plant of the invention belonging to a previous generation, then obtaining a transgenic progeny plant of the invention belonging to a new generation by growing up the transgenic seed;
   and/or
propagating clonally a transgenic plant of the invention belonging to a previous generation to give a transgenic progeny plant of the invention belonging to a new generation;
   and/or
crossing a first-generation a transgenic plant of the invention belonging to a previous generation with another compatible plant to give a transgenic progeny plant of the invention belonging to a new generation;
   and optionally;
obtaining transgenic progeny plants of one or more further generations from the progeny plant thus obtained.

These techniques may be used in any combination. for example, clonal propagation and sexual propagation may be used at different points in a process that gives rise to a transgenic plant suitable for cultivation. In particular, repetitive back-crossing with a plant taxon with agronomically desirable characteristics may be undertaken. Further steps of removing cells from a plant and regenerating new plants therefrom may also be carried out.

Also, further desirable characteristics may be introduced by transforming the cells, plant tissues, plants or seeds, at any suitable stage in the above process, to introduce desirable coding sequences other than the polynucleotides of the invention. This may be carried out by the techniques described herein for the introduction of polynucleotides of the invention.

For example, further transgenes may be selected from those coding for herbicide resistance traits; other insect resistance traits, notably genes encoding *Bacillus thuringiensis* (*Bt*) toxins; or virus-resistant traits.

### Transgenic plant cells, plant parts and tissues, plants and seeds of the invention

The invention also provides transgenic plant cells, plant parts and tissues, plants and seeds. These are typically obtainable, or obtained, by the methods described above. They may be of any botanical taxon, e.g. any species or lower taxonomic grouping. Preferably, they are of a crop plant species.

Transgenic plant cells, plant parts and tissues, plants and seeds of the invention may thus be of a monocotyledonous (monocot) or dicotyledonous (dicot) taxon. Preferred dicot crop plants include solanaceous crops such as tomato, potato and tobacco; sugarbeet; cruciferous crops, including oilseed rape; linseed; tobacco; sunflower; fibre crops such as cotton; and leguminous crops such as peas, beans, e.g. soybean, and alfalfa. Suitable monocots include graminaceous plants such as wheat, maize, rice, oats, barley and rye, sorghum, triticale and sugar cane.

Typically, a polypeptide of the invention is expressed in a plant of the invention. depending on the promoter used, this expression may be constitutive or inducible. Similarly, it may be tissue- or stage-specific, i.e. directed towards a particular plant tissue or stage in plant development.

Preferably, plant cells, plant parts and tissues, plants and seeds of the invention exhibit insect resistance due, at least in part, to expression of a polypeptide of the invention.

### Uses of polypeptides and plants of the invention

Polypeptides of the invention are useful in conferring insect resistance on plants. One embodiment of the invention is to apply the polypeptides of the invention. Another is to provide a transgenic plant of the invention which expresses a polypeptide of the invention *in situ*. Thus, the invention provides methods of controlling insects on plants, preferably crop plants. Desirably, the insects suffer increased mortality or are completely eliminated. For example, then, the invention provides method for controlling insects on plants at a locus where such plants are being cultivated.

## Claims

1. Use of a sporamin polypeptide to control an insect pest on a plant.

2. Use according to claim 1 wherein the polypeptide has trypsin inhibitor activity.

3. Use according to claim 1 or 2 wherein the polypeptide has trypsin inhibitor activity, and is encoded by a polynucleotide comprising a coding sequence selected from:
(a) the nucleotide sequence of Fig 1 (SEQ ID No. 1), or a coding sequence within the sequence of Fig 1 beginning with ATG at nucleotides 56 to 58, or the complement of either of those sequences; or
(b) a nucleotide sequence capable of hybridising selectively to the coding sequence of (a); or
(c) a nucleotide sequence that is degenerate as a result of the genetic code to a nucleotide sequence of (a) or (b); or
(d) a nucleotide sequence having at least 80% homology to a sequence of (a), (b) or (c).

4. Use according to claim 3 wherein the polypeptide is encoded by the sequence beginning with ATG at nucleotides 56 to 58 of Fig 1 (SEQ ID No. 1) and ending with TAA at nucleotides 713 to 715 of Fig 1 (SEQ ID No. 1).

5. Use according to any one of the preceding claims comprising applying the polypeptide to the plant.

6. Use according to any one of claims 1 to 4 comprising providing a transgenic plant which expresses the polypeptide.

7. A polynucleotide encoding a sporamin protein having trypsin inhibitor activity, which polynucleotide comprises a coding sequence selected from:
(a) the nucleotide sequence of Fig 1 (SEQ ID No. 1), or a coding sequence within the sequence of Fig 1 beginning at ATG at nucleotides 56 to 58, or the complement of either of those sequences; or
(b) a nucleotide sequence capable of hybridising selectively to the coding sequence of (a); or
(c) a nucleotide sequence that is degenerate as a result of the genetic code to a nucleotide sequence of (a) or (b); or
(d) a nucleotide sequence having at least 80% homology to a sequence of (a), (b) or (c).

8. A polynucleotide according to claim 7 which is a DNA.

9. A polynucleotide according to claim 7 or 8 wherein the coding sequence encodes the polypeptide encoded by the sequence beginning with ATG at nucleotides 56 to 58 of Fig 1 (SEQ ID No. 1) and ending with TAA at nucleotides 713 to 715 of Fig 1 (SEQ ID No. 1).

10. A polypeptide encoded by a polynucleotide sequence as defined in any one of claims 7 to 9.

11. A chimeric gene comprising a polynucleotide according to any one of claims 7 to 10 operably linked to regulatory sequences that allow expression of the coding sequence in a host cell.

12. A chimeric gene according to claim 11 wherein the regulatory sequences allow expression of the coding sequence in a plant cell.

13. A vector comprising a polynucleotide according to any one of claims 6 to 9 or a chimeric gene according to claim 11 or 12.

14. A vector according to claim 13 which is an expression vector.

15. A vector according to claim 13 or 14 which is a binary vector.

16. A vector according to claim 15 which is vector pBI 121

17. A cell transformed or transfected with a vector according to any one of claims 13 to 16.

18. A cell according to claim 17 which is a prokaryotic cell or a plant cell.

19. A cell according to claim 17 or 18 having the chimeric gene integrated into its genome.

20. A cell according to claim 17, 18 or 19 which is a transformant bacterium.

21. A bacterium according to claim 20 which is an *Agrobacterium tumefaciens* bacterium.

22. A process for the production of a polypeptide according to claim 10, which process comprises:
(a) cultivating a cell according to any one of claims 17 to 21 under conditions that allow the expression of the polypeptide; and
(b) recovering the expressed polypeptide.

23. A method of obtaining a transgenic plant cell comprising:
(a) transforming a plant cell with a vector according to any one of claims 13 to 16 to give a transgenic plant cell,

24. A method according to claim 23 wherein transformation is effected via an *Agrobacterium* as defined in claim 21.

25. A method of obtaining a first-generation transgenic plant comprising:
(b) regenerating a transgenic plant cell produced by the method of claim 23 or 24 to give a transgenic plant.

26. A method of obtaining a transgenic plant seed comprising:
(c) obtaining a transgenic seed from the transgenic plant produced by step (b) of the method of claim 25.

27. A method of obtaining a transgenic progeny plant comprising obtaining a second-generation transgenic progeny plant from a first-generation transgenic plant produced by the method of claim 26, and optionally obtaining transgenic plants of one or more further generations from the second-generation progeny plant thus obtained.

28. A method according to claim 27 comprising:
(c) obtaining a transgenic seed from the first-generation transgenic plant produced by the method of claim 25, then obtaining a second-generation transgenic progeny plant from the transgenic seed;
and/or
(d) propagating clonally the first-generation transgenic plant produced by the method of claim 25 to give a second-generation progeny plant;
and/or
(e) crossing a first-generation transgenic plant produced by the method of claim 25 with another plant to give a second-generation progeny plant;
and optionally;
(f) obtaining transgenic progeny plants of one or more further generations from the second-generation progeny plant thus obtained.

29. A transgenic plant cell, first-generation plant, plant seed or progeny plant obtainable by a method according to any one of claims 23 to 28.

30. A transgenic plant, or plant seed comprising plant cells according to any one of claims 17 to 19.

31. A transgenic plant cell callus comprising plant cells according to any one of claims 17 to 19, or obtainable from a transgenic plant cell, first-generation plant, plant seed or progeny plant according to claim 29.
